# EUROPÄISCHE PATENTSCHRIFT

(11) **EP 1 200 036 B1**
(45) Veröffentlichungstag und Bekanntmachung des Hinweises auf die Patenterteilung: **14.01.2004**
(21) Anmeldenummer: 00954572.4
(22) Anmeldetag: 28.07.2000
(51) Int. Cl.: A61K 6/00, A61K 6/083, A61K 6/087

(54) **ADHÄSIVSYSTEME**
ADHESIVE SYSTEMS
SYSTEMES ADHESIFS

(30) Priorität: 06.08.1999 DE 19937091
(43) Veröffentlichungstag der Anmeldung: 02.05.2002
(73) Patentinhaber: 3M ESPE AG, 82229 Seefeld (DE)
(72) Erfinder: LUCHTERHANDT, Thomas, D-86926 Greifenberg (DE); GUGGENBERGER, Rainer, D-82211 Herrsching (DE); GRUPP, Hendrik, M., D-82205 Gilching (DE)
(86) Internationale Anmeldenummer: PCT/EP2000/007272
(87) Internationale Veröffentlichungsnummer: WO 2001/010388

(56) Entgegenhaltungen:
- EP-A- 0 712 622
- EP-A- 0 897 710
- WO-A-00/56800
- WO-A-98/46197
- DE-A- 19 648 283

## Beschreibung

Die vorliegende Erfindung betrifft Adhäsivsysteme auf Basis von kationisch härtenden Verbindungen und ihre Verwendung.

In polymerisierbaren Dentalmassen wurden bislang vorwiegend Methacrylat- und Acrylatmonomere verwendet. Besondere Aufmerksamkeit verdient das von Bowen beschriebene 2,2-Bis-[4-(2-hydroxy-3-methacryloyloxypropoxy)phenyl]-propan (Bis-GMA) [US-A-3 066 112]. Mischungen dieses Methacrylats mit Triethylenglykol-dimethacrylat dienen auch heute noch als Standardrezeptur für dentale plastische Direkt-Füllungswerkstoffe. Auch Methacrylderivate des zweifach formylierten Bis-(hydroxymethyl)tricyclo[5.2.1.0^{2,6}]decans haben sich als Monomere für Dentalcomposite bewährt [W. Gruber et al., DE-A-27 14 538; W. Schmitt et al., DE-A-28 16 823; J. Reiners et al., EP-A-0 261 520]. Ein wesentlicher Nachteil dieser Dentalmassen ist aber der durch die Polymerisation auftretende hohe Volumenschrumpf. Dieser kann beispielsweise durch den Einsatz von ringöffnenden Monomeren, wie den kationisch härtenden Epoxiden, minimiert werden.

Über kationisch härtbare Epoxidmassen für dentale Anwendungen ist nur wenig bekannt: Die US-A-5 556 896 beschreibt epoxidhaltige Massen, die notwendigerweise als schrumpfkompensierende Monomere Spiroorthocarbonate enthalten müssen. Bowen beschreibt eine Masse, enthaltend Quarzsand und ein aliphatisches Diepoxid (Bisphenol-A-diglycidylether), die im ausgehärteten Zustand angeblich gute Stabilität im Mundmilieu aufweist [J. Dent. Res. 35, 1956, 360-379]. In der AT-A-204 687 werden Epoxid-Dentalmassen auf Basis von Bisphenol-A, die mittels Lewis-Säure-Katalysatoren ausgehärtet werden, beschrieben. Die Schriften DE-A-196 48 283, WO-96/13538 und WO-95/30402 beschreiben ebenfalls polymerisierbare Dentalmassen auf Basis von Epoxiden und deren Verwendung.

Obwohl es umfangreiche Erfahrungen mit Epoxiden und cycloaliphatischen Epoxiden gibt (US-A-2 716 123, US-A-2 750 395, US-A-2 863 881, US-A-3 187 018), sind solche Monomere und daraus formulierte kationisch polymerisierbare Massen mit den für dentale Anwendungen notwendigen Eigenschaften zu keinem Zeitpunkt kommerziell verfügbar gewesen.

Ursache dafür ist die Tatsache, dass die Aushärtung dieser kationisch polymerisierenden Massen durch Wasser inhibiert wird und die Zahnhartsubstanz, beispielsweise im Dentin, ca. 11 bis 16 Gewichtsprozent Wasser enthält (G.-H. Schumacher et. al., Anatomie und Biochemie der Zähne, Gustav Fischer Verlag, 1990, 4. Auflage). So ist es leicht zu erklären, dass beispielsweise Formulierungen von dentalen Füllungsmaterialien auf Epoxidbasis keine Eigenhaftung auf Dentin zeigen können und so die Verwendung solcher Materialien nicht möglich war.

Zur Befestigung von dentalen Füllungsmaterialien auf (Meth-)Acrylatbasis - also radikalisch härtenden Systemen - werden sog. Adhäsivsysteme verwendet.

Die Qualität dieser Adhäsivsysteme spiegelt sich in den folgenden Kriterien wieder:
- Vollständige Haftung an der Zahnhartsubstanz ohne Fehlstellen ("Versiegelung");
- Vollständige Haftung an dem Füllungsmaterial,
- Dauerhafter Verbund.

Eine Aushärtung von kationisch vernetzenden Systemen auf der Zahnhartsubstanz ist wegen den zuvor genannten Umständen nicht zu erwarten. Auf dem Markt ist zu keiner Zeit ein Adhäsivsystem auf kationisch vemetzender Basis für (auch) kationisch härtende Massen kommerziell verfügbar gewesen.

Die DE-A-197 43 564 beschreibt zwar strahlenhärtbare Haftvermittler - sog. Primer - Zusammensetzungen auf der Basis von lösungsmittelfreien, kationisch und/oder radikalisch härtbaren Vernetzungssystemen, doch werden diese nur zur Beschichtung von wasserfreien Materialien, beispielsweise Kunststoffen, wie Polyvinylidenchlorid (PVDC) oder Silikon, verwendet.

Die WO-98/47046 beschreibt photopolymerisierbare Mischungen auf Epoxidbasis, enthaltend ein Epoxidharz, ein lodoniumsalz, ein im sichtbaren Licht sensibles Übertragungsmolekül und einen Elektronendonor und beansprucht deren Verwendung als dentales Adhäsivsystem. Es zeigt sich jedoch, dass mit solchen Mischungen auf der Zahnhartsubstanz keine Haftung zu kationisch härtenden Mischungen zu erzielen ist (siehe Vergleichsmischungen 1 bis 3 der vorliegenden Schrift).

Die WO-99/34766 führt aus, dass Zusammensetzungen mit einem hohen Anteil an kationisch härtbaren Gruppen nicht oder nur sehr schlecht auf Zahnhartgewebe haften. Zur Lösung des Problems wird vorgeschlagen entweder eine Hybrid-Zusammensetzung, enthaltend Bestandteile mit radikalisch und kationisch polymerisierbaren Gruppen, oder eine Zusammensetzung, die weitgehend frei ist von kationisch polymerisierbaren Gruppen, bereitzustellen.

Die WO 98/46197 A1 betrifft Dentalzusammensetzungen mit verbesserten Eigenschaften. Die Zusammensetzungen weisen u. a. neben einer polymerisierbaren Komponente, bei der es sich auch um eine kationisch polymerisierbare Komponente handeln kann, einen mit Säure reaktiven Füllstoff und eine saure Komponente auf, wobei die Menge der eingesetzten Bestandteile derart ist, dass die Zusammensetzung über bestimmte rheologische Eigenschaften verfügt. Bei den mit Säure reaktiven Füllstoffen handelt es sich z.B. um Calcium- oder Magnesiumoxid. Es wird ausgeführt, dass die bevorzugten Materialien keine Klebrigkeit aufweisen und dass Klebrigkeit und leichtes Fließvermögen unerwünscht sind.

Eine primäre Aufgabe der vorliegenden Erfindung ist es, ein alternatives Adhäsivsystem zur Verfügung zu stellen, das die oben genannten Probleme löst und vorzugsweise auf Wasser enthaltenden Hartgewebe, wie Zahn, zur Haftung gebracht werden kann.

Erfindungsgemäß wird diese Aufgabe gelöst durch Verwendung mindestens einer Komponente i) die fähig ist, eine kationische Polymerisation zu starten und gewählt ist aus, ungesättigten Carbonsäuren und deren Anhydriden oder Säurechloriden, ungesättigten organischen Phosphorsäuren und deren Estern, ungesättigten organischen Phosphonsäuren und deren Estern, ungesättigte organischen Sulfonsäuren und deren Estern, und mindestens einer Komponente ii), die kationisch polymerisierbar ist, zur Herstellung eines Adhäsivsystems.

Überraschenderweise wurde gefunden, dass bei der Verwendung von kationisch polymerisierbaren Adhäsivsystemen mit Photopolymerisationsinitiatoren, wie sie in der WO-98/47046 beschrieben sind, zur Befestigung von kationisch härtenden Materialien auf Wasser enthaltenden .Hartgewebe, eine ausreichende Haftung nicht zu erzielen ist (siehe Vergleichsmischungen 1 bis 3 der vorliegenden Schrift).

Dies gelingt nur, wenn Adhäsivsysteme verwendet werden, die die oben erwähnten und im Nachfolgenden beschriebenen, speziellen Säuren enthalten.

Zusätzlich können die in der genannten WO-98/47046 beschriebenen Photopolymerisationsinitiatoren, bestehend aus einem lodoniumsalz, einem im sichtbaren Licht sensiblen Übertragungsmolekül und einem Elektronendonor, sowie beispielsweise die in der DE-A-197 36 471 und DE-A-197 43 564 beschriebenen Initiatoren für die kationische Polymerisation, enthalten sein.

Des weiteren wurde gefunden, dass eine besonders vorteilhafte Methode zur Befestigung genannter Materialien auf genanntem Hartgewebe darin besteht, als Säuren radikalisch vernetzbare Materialien zu verwenden und zusätzlich eine radikalische Vernetzung durchzuführen. So bleiben die kationisch vernetzbaren Gruppen für eine Copolymerisation mit dem Füllungsmaterial in der Grenzschicht größtenteils erhalten.

Ein weiterer für die Haftung förderlicher Effekt dieser Reaktionsführung, ist die Ausbildung der bei radikalisch vernetzenden Systemen bekannterweise entstehenden "Schmierschicht". Diese kommt durch die Sauerstoffinhibierung zustande und sorgt für eine optimale Vermischung in der Grenzfläche zwischen Adhäsiv und Füllungssystem.

Im Folgenden wird die Erfindung näher beschrieben.

Die Begriffe "enthalten" und "umfassen" im Sinne der Erfindung leiten eine nichtabschließende Aufzählung von Merkmalen ein.

Die adhäsiven Mischungen, welche die beschriebenen Vorteile bei erfindungsgemäßer Verwendung aufweisen, enthalten als Bestandteil i) vorzugsweise 0,01 bis 95 Gew.-%, insbesondere 0,1 bis 90 Gew.-% und besonders bevorzugt 0,1 bis 80 Gew.-% eines Initiatorsystems, das aus den oben beschriebenen speziellen Säuren gewählt ist,
und als Bestandteil ii) vorzugsweise 5 bis 99,99 Gew.-%, insbesondere 10 bis 99,90 Gew.-% und besonders bevorzugt 20 bis 99,90 Gew.-% eines kationisch polymerisierbaren Materials.

In einer bevorzugten Ausführungsform sind die Säuren selbst radikalisch polymerisierbar, und die Gesamtmischung enthält zusätzlich einen Initiator, der befähigt ist, die radikalische Polymerisation zu starten.

Als Komponente i) kommen folgende kationische Initiatoren in Betracht:
- Ungesättigte Carbonsäuren, wie ein- oder mehrfach ungesättigte organische Mono-, Di- oder Polycarbonsäuren, oder deren Vorstufen, die mit Wasser Säuren bilden können, wie Anhydride oder Säurechloride, insbesondere auch saure Methacrylsäureester oder Amide.
   Bevorzugt sind ungesättigte Carbonsäuren der folgenden Formel: 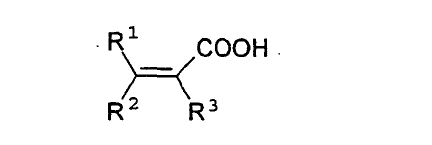 in welcher bedeuten:
   R¹, R²,R³ = H, C₁- bis C₂₅- Alkyl- oder Cycloalkylreste, ggf. substituiert mit N, O, S, Si, P oder Halogen, oder aromatische C₆ bis C₁₂-Reste oder heterocyclische C₃ bis C₁₂-Reste mit N, O, S, P und ggf. substituiert mit Halogen.
   Ebenso können Säuren, wie die 4-Methacryloxyethyltrimellitsäure oder ihre Anhydride (Takeyama, M. et al., J.Jap.Soc. f. Dent. App. A. Mat. 19, 179 (1978)), oder die Umsetzungsprodukte von Trimelitsäurechloridanhydrid mit aminischen, thiolischen oder hydroxylischen (Meth-)Acrylsäureestern, wie 2-Hydroxyethylenmethacrylat oder Methacroyloxy-ethyl-o-phthalat, verwendet werden.
- Ungesättigte organische Phosphor- und Phosphonsäuren.
   Bevorzugt sind beispielsweise ungesättigte organische Ester der Monofluorphosphonsäuren, wie sie in der US-A-3 997 504 beschrieben sind, ungesättigte organische Ester von Säuren des Phosphors, die Chlor oder Brom direkt am Phosphor gebunden enthalten, wie sie in der EP-A-0 058 483 beschrieben sind, ungesättigte organische Ester der Phosphorsäure, die als cyclische Pyrophosphate (Anhydride) vorliegen, wie sie in der DE-A-3 048 410 beschrieben sind und ungesättigte organische Ester von Phosphor- oder Phosphonsäuren, wie sie in den DE-A-2 711234 und DE-A-3 150 285 beschrieben sind. Genauso bevorzugt sind die hydrolysestabilen, polymerisierbaren Acrylphosphonsäuren der DE-A-19 746 708.
- Weiterhin sind geeignet die phosphorsäuregruppenhaltigen Monomere, wie sie in den US-A-4 182 035, US-A-4 222 780, US-A-4 235 633, US-A-4 359 117 und US-A-4 368 043 sowie in der EP-A-0 084 407 beschrieben sind. Bevorzugt werden in den erfindungsgemäßen Adhäsivmischungen ethylenisch ungesättigte Phosphorsäureester gemäß folgender Formel verwendet: 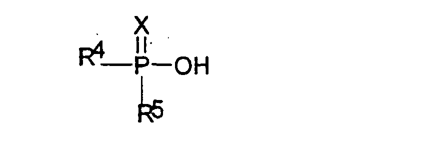
in welcher bedeuten:
X = O, S;
R⁴ und R⁵ unabhängig voneinander H, OH, oder C₁ bis C₂₅-Alkyl oder Cycloalkyl, ggf. substituiert oder verbrückt mit Heteroatomen wie N, Halogen, Si, O, oder S, aromatische C₆ bis C₁₂ -, oder/und heterocyclische C₄ bis C₁₂-Reste, oder substiuiert mit Acrylsäureestern, wobei die Reste R⁴ und R⁵ auch unabhängig voneinander über O an den Phosphor gebunden sein können,
oder 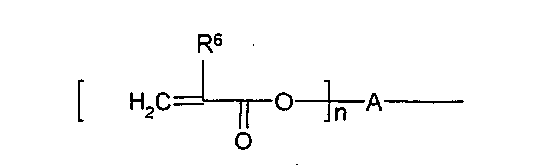 ,wobei
R⁶ Wasserstoff oder C₁- bis C₆-Alkyl bedeutet,
n eine ganze Zahl ≥ 1 ist, und
A einen zweiwertigen C₁- bis C₂₅- Alkylen- oder Cycloalkylenrest, ggf. substituiert oder verbrückt mit N, O, S, Si, P oder Halogen, oder einen aromatischen C₆ bis C₁₂ - Rest oder/und heterocyclischen C₄ bis C₁₂ -Rest mit N, O, S, oder P und ggf. substituiert mit Halogen, bezeichnet, mit der Maßgabe, dass der Rest, der R⁶ enthält, mindestens einmal vorhanden ist. - Substituierte, ungesättigte organische Sulfonsäuren und deren Ester.

Die vorher genannten kationischen Initiatorsysteme können alleine oder auch in Mischungen eingesetzt werden, wobei die Aufzählungen beispielhaft und in keiner Weise abschließend zu verstehen sind.

Als Komponente ii) können in den erfindungsgemäßen Adhäsivmischungen kationisch härtende Materialien verwendet werden, die aus folgenden Gruppen gewählt sind. Dabei ist auch diese Aufzählung beispielhaft und nicht abschließend zu verstehen.
- Epoxidharze, wie sie in der DE-A-196 48 283 A1 beschrieben sind, insbesondere 1,3,5,7-Tetrakis-(2,1-ethandiyl-3,4-epoxycyclohexyl)-1,3,5,7-tetramethylcyclotetrasiloxan.
- Spiro-ortho-carbonate, wie 3,9-Diethyl-3,9-dipropionyloxymethyl-1,5,7,11-tetraoxaspiro-[5.5]undecan, 2,8-Dimethyl-1,5,7,11-tetraoxaspiro-[5.5]undecan, oder 5,5-Diethyl-19-oxadispiro[1,3-dioxan-2-2'-1,3-doixan-5',4"-bicyclo[4.1.0]heptan, wie sie in der US-A-5 556 896 beschrieben sind, oder Spiro-ortho-ester, oder Oxetane, wie sie in der DE-A-197 36 471 S.3 f. beschrieben sind oder Vinylether, wie Alkyl- oder Cycloalkylvinylether, Ethylenglycoldivinylether, Triethylenglycoldivinylether, Glycidylvinylether oder Butandiolvinylether.

Die vorher genannten Materialien können allein oder auch in Mischungen eingesetzt werden.

Außerdem können gegebenenfalls radikalisch härtende Verbindungen, die keine Säuregruppen enthalten, zugemischt werden, vorzugsweise in einer Menge von weniger als 25 Gew.-%. Diese Ausführungsform führt zur Bildung eines interpenetrierenden Netzwerks.

Eine im Verhältnis zu der Menge an kationisch polymerisierbaren Verbindungen geringere Menge an radikalisch polymerisierbaren Verbindungen stellt sicher, dass die Matrixeigenschaften des Netzwerkes aus kationisch polymerisierten Verbindungen die Eigenschaften des Adhäsivsystems überwiegend bestimmen.

Typische Monomere bzw. Präpolymere, die nach dem Radikalketten-mechanismus aushärten und in der Komponente ii) vorhanden sein können, sind Acrylate oder Methacrylate. Geeignet sind beispielsweise ein- und mehrfunktionelle (Meth)acrylatmonomere. Typische Vertreter dieser Verbindungs-klasse (DE-A-4 328 960) sind Alkyl(meth)acrylate, einschließlich der Cycloalkyl(meth)acrylate, Aralkyl(meth)acrylate und 2-Hydroxyalkyl(meth)acrylate, beispielsweise Hydroxypropylmethacrylat, Hydroxyethylmethacrylat, Isobornyl-acrylat, Isobornylmethacrylat, Butylglycolmethacrylat, Acetylglykolmethacrylat, Triethylenglycoldimethacrylat, Polyethylenglycoldimethacrylat, 2-Phenylethylmeth-acrylat, 2-Ethylhexylmethacrylat, Cyclohexylmethacrylat, Laurylmethacrylat und Hexandioldi(meth)acrylat. Verwendet werden können auch die langkettigen Monomere, wie sie in der US-A-3 066 112 beschrieben sind, auf der Basis von Bisphenol-A und Glycidylmethacrylat oder deren durch Addition von Isocyanaten entstandene Derivate. Geeignet sind auch Verbindungen des Typs Bisphenyl-Adiethyloxy(meth)acrylat und Bisphenol-A-dipropyloxy(meth)acrylat. Weiterhin können die oligoethoxylierten und oligopropoxylierten Bisphenol-A-diacryl- und - dimethacrylsäureester Verwendung finden. Gut geeignet sind außerdem die in der DE-A-2 816 823 genannten Diacryl- und Dimethacrylsäureester des Bis(hydroxymethyl)-tricyclo[5.2.1.0^{2,6}]-decans und die Diacryl- und Dimethacryl-säureester der mit 1 bis 3 Ethylenoxid- und/oder Propylenoxideinheiten verlängerten Verbindungen des Bis(hydroxymethyl)-tricyclo [5.2.1.0^{2,6}]-decans. Es können auch Mischungen der genannten Monomeren verwendet werden.

Sind zusätzlich ethylenisch ungesättigte Gruppen in der Komponente i) enthalten oder radikalisch polymerisierbare Verbindungen zugemischt, so können auch obligatorisch zusätzlich radikalische Initiatoren verwendet werden.

Die radikalbildenden Initiatoren, welche in den Mischungen enthalten sein können, sind in der Literatur beschrieben (z. B. J.-P Fouassier, Photoinitiation, Photopolymerization and Photocuring, Hanser Publishers, Munich, Vienna, New York, 1995 oder auch J.-P Fouassier, J. F. Rabek (Hrsg.), Radiation Curing in Polymer Science and Technology, Vol. II, Elsevier Apllied Science,, London, New York, 1993). Sie können durch UV- oder sichtbares Licht aktivierbare Substanzen sein, wie Benzoinalkylether, Benzilketale, Acylphosphinoxide oder aliphatische und aromatische 1,2-Diketonverbindungen, beispielsweise Campherchinon, wobei die Katalysatoraktivität durch Zusatz von Aktivatoren, wie tertiären Aminen oder organischen Phosphiten, in an sich bekannter Weise beschleunigt werden kann.

Geeignete Initiatorsysteme zur Auslösung der radikalischen Polymerisation über einen Redox-Mechanismus sind beispielsweise die Systeme Peroxid/Amin, Peroxid/Barbitursäurederivate oder Peroxid/Säuren u. dergl. Bei Verwendung solcher Initiatorsysteme ist es zweckmäßig, einen Initiator (z. B. Peroxid) und eine Katalysatorkomponente (z. B. Amin) getrennt bereitzuhalten. Die beiden Komponenten werden dann kurz vor ihrer Anwendung miteinander homogen gemischt.

Die erfindungsgemäßen Adhäsivsysteme können auch Füllstoffe, Farbstoffe, Fließmodifikatoren, Stabilisatoren, Lösungsmittel, ionenabgebende Substanzen, bakterizid oder antibiotisch wirksame Substanzen, die Röntgenopazität erhöhende Verbindungen oder weitere Modifikatoren enthalten.

Als Verdünnungsmittel sind bevorzugt Lösungsmittel, wie Dialkylketone (z. B. Aceton, Methyl-ethylketon), Acetylaceton oder Alkohole (z. B. Ethanol, Propanol) oder auch dünnfließende polymerisierbare Substanzen wie 2-Hydroxyethylmethacrylat oder (2,3-Epoxypropyl)-methacrylat.

Als Füllstoffe sind beispielsweise Stoffe geeignet, wie sie in üblichen Dentalmaterialien verwendet werden, besonders bevorzugt Quarz, Aerosile, hochdisperse Kieselsäuren, organische Füllstoffe oder Glas oder Mischungen dieser Stoffe oder auch solche wie sie in der DE-A-196 48 283 A1 (Seite 10, Zeile 48 bis 59) beschrieben sind.

Bei den ionenabgebenden Substanzen sind solche bevorzugt, die die Freisetzung von Fluoridionen ermöglichen, wie Fluoridsalze der ersten oder zweiten Hauptgruppe, wie Natriumfluorid oder Calciumfluorid, oder komplexe Fluoridsalze, wie KZnF₃, oder wie sie in der EP-A-0 717 977 beschrieben werden, Fluorid abgebende Gläser, sowie Mischungen dieser Fluroridionenquellen.

Außerdem können zusätzlich eine oder mehrere nicht polymerisierbare Säuren enthalten sein, wie eine Carbonsäure, Lewissäure oder Komplexsäure.

Als bakterizid oder antibiotisch wirksame Substanzen können beispielsweise Chlorhexidin, Pyridinumsalze oder die üblichen pharmazeutischen Substanzen, wie β-Lactamantibiotika (Penicilline), Cephalosporine, Tetracycline, Ansamycine, Kanamycine, Chloramphenicol, Fosfomycin, antibakterielle Makrolide, Polypeptid-Antibiotika, Chemotherapeutika, wie Sulfonamide, Dihydrofolatreduktase-Hemmstoffe, Nitrofuran-Derivate oder Gyrasehemmer verwendet werden.

Enthalten die adhäsiven Mischungen über die Komponenten i) und ii) hinaus Zusatzstoffe, so können diese in den Mengen von 0,1 Gew.-% bis 90 Gew.-% einzeln oder gemischt vorhanden sein, wobei die Mischung so zubereitet werden, dass sie sich mit den Komponenten i) und ii) zusammen insgesamt zu 100 Gew.-% ergänzen.

Selbstverständlich können die erfindungsgemäßen Adhäsivmischungen auch für die Befestigung von rein radikalisch härtenden Materialien verwendet werden.

Im folgenden wird die Erfindung anhand von Beispielen näher beschrieben, wobei diese als Ausführungsbeispiele und in keiner Weise limitierend zu verstehen sind.

Die Haftmessungen wurden auf Rinderdentin durchgeführt und sind auf Rinderschmelz noch wesentlich höher.

### Haftmessung an Rinderzähnen durch adhäsive Befestigung eines Füllungsmaterials:

Die Überprüfung des Haftverbundes erfolgte durch einen Haftabzugsversuch an Rinderzähnen. Pro Versuch wurden 5 frisch extrahierte Rinderzähne mittels Schleifpapier soweit abgeschliffen, dass eine ausreichend große freiliegende Dentinfläche entstand. Auf diese Flächen wurden jeweils Wachsplättchen mit einem ausgestanzten Loch von 6 mm geklebt, um eine standardisierte Haftfläche zu erhalten. Dann wurde die Prüffläche in Anlehnung an das praxisübliche Vorgehen mittels einer üblichen Phosphorsäurelösung (Ätzgel Minitip®, Fa. ESPE Dental AG, Seefeld) für 20 Sekunden angeätzt und anschließend mit Wasser abgespült. Auf die derart vorbereiteten Dentinflächen wurde eine zur vollständigen Benetzung der Prüfoberfläche ausreichende Menge der Versuchsmischungen mit einem Microbrush 20 sec. lang eingearbeitet, kurz mit Druckluft verblasen und mittels eines Lichtpolymerisationsgerätes (Elipar Highlight®, Fa. ESPE) für 20 Sekunden polymerisiert. Anschließend wurde das kationisch härtende Füllungsmaterial (entweder das kationische Füllungsmaterial, dessen Herstellung nachstehend beschrieben ist, oder handelsübliches Pertac II (ESPE Dental AG, Seefeld)) in die Aussparungen der Wachsplättchen eingebracht und durch 40 sec. Belichtung auspolymerisiert. Das Wachsplättchen wurde entfernt und die Prüfkörper 24 h bei 36°C und 100% Luftfeuchtigkeit gelagert. Dann wurden die Prüfkörper in einem Zugversuch (Zwick Universalprüfmaschine) abgezogen.

Die dabei ermittelten Haftwerte sind Tabelle 1 zu entnehmen.

### Herstellung eines kationisch härtenden Füllungsmaterials:

In einem Dreifingerkneter werden die folgenden Bestandteile zu einer homogenen Paste geknetet. Man verwendet auf 100 g Paste:
- 75,000 Gew.-% (75,000 g) Quarz (mittlere Korngröße 0,9 µm, wurde mit 5 Gew.-% Glycidyloxypropyltrimethoxysilan silanisiert);
- 0,525 Gew.-% (0,525 g) 4-Methylphenyl-4-isopropylphenyl-iodoniumtetrakis-(penta-fluorophenyl)borat;
- 0,223 Gew.-% (0,223 g) Campherchinon (Fa. Merck, Darmstadt);
- 0,001 Gew.-% (0,001 g) Ethyl-4-dimethylaminobenzoat (Fa. Merck, Darmstadt);
- 0,001 Gew.-% (0,001 g) 2-Butoxyethyl-4-dimethylaminobenzoat;
- 12,125 Gew.-% (12,125 g) 3,4-Epoxycyclohexyl-3,4-epoxycyclohexancarboxylat;
- 12,125 Gew.-% (12,125 g) 1,3,5,7-Tetrakis-(2,1-ethandiyl-3,4-epoxycyclohexyl)-1,3,5,7-tetramethylcyclotetrasiloxan.

### Herstellung der erfindungsgemäßen Adhäsivmischung 1:

Zur Herstellung von 10 g der Adhäsivmischung 1 werden die folgenden Bestandteile intensiv miteinander vermischt:
- 48,650 Gew.-% (4,865 g) 1,3,5,7-Tetrakis-(2,1-ethandiyl-3,4-epoxycyclohexyl)-1,3,5,7-tetramethylcyclotetrasiloxan;
- 38,920 Gew.-% (3,892 g) 10-Methacryloyloxyethylphosphat;
- 9,380 Gew.-% (0,938 g) 2-Hydroxyethylmethacrylat;
- 2,000 Gew.-% (0,200 g) Rhodorsil PI 2074 (Fa. Rhone Poulenc, lodoniumsalz);
- 0,600 Gew.-% (0,060 g) Campherchinon (Fa. Merck, Darmstadt);
- 0,450 Gew.-% (0,045 g) Ethyl-4-dimethylaminobenzoat (Fa. Merck, Darmstadt);

### Herstellung der erfindungsgemäßen Adhäsivmischung 2:

Zur Herstellung von 10 g der Adhäsivmischung 1 werden die folgenden Bestandteile intensiv miteinander vermischt:
- 48,650 Gew.-% (4,865 g) 3,4-Epoxycyclohexyl-3,4-epoxycyclohexancarboxylat;
- 38,920 Gew.-% (3,892 g) 10-Methacryloyloxyethylphosphat;
- 9,380 Gew.-% (0,938 g) 2-Hydroxyethylmethacrylat;
- 2,000 Gew.-% (0,200 g) Rhodorsil PI 2074 (Fa. Rhone Poulenc, lodoniumsalz);
- 0,600 Gew.-% (0,060 g) Campherchinon (Fa. Merck, Darmstadt);
- 0,450 Gew.-% (0,045 g) Ethyl-4-dimethylaminobenzoat (Fa. Merck, Darmstadt);

### Referenz - Adhäsivmischung 3:

Bei diesem Versuch wurde nicht mit einer üblichen Phosphorsäurelösung (Ätzgel Minitip®, Fa. ESPE Dental AG, Seefeld) für 20 Sekunden angeätzt, sondern eine zur Benetzung der Oberfläche ausreichende Menge an 1,3,5,7-Tetrakis-(2,1-ethandiyl-3,4-epoxycyclohexyl)-1,3,5,7-tetramethylcyclotetrasiloxan mit einem Microbrush 20 sec. lang einmassiert, kurz mit Druckluft verblasen und dann eine 5 gewichtsprozentige Lösung aus HSbF₆ in Ethanol aufgetragen und mit Druckluft verblasen. Anschließend wurde das kationisch härtende Füllungsmaterial in die Aussparungen der Wachsplättchen eingebracht und durch 40 sec. Belichtung auspolymerisiert. Das Wachsplättchen wurde entfernt und die Prüfkörper 24 h bei 36°C und 100% Luftfeuchtigkeit gelagert. Dann wurden die Prüfkörper in einem Zugversuch (Zwick Universalprüfmaschine) abgezogen.

### Herstellung der Vergleichsmischung 1:

Zur Herstellung von 10 g der Vergleichsmischung 1 werden die folgenden Bestandteile intensiv miteinander vermischt:
- 97,300 Gew.-% (9,730 g) 1,3,5,7-Tetrakis-(2,1-ethandiyl-3,4-epoxycyclohexyl)-1,3,5,7-tetramethylcyclotetrasiloxan;
- 2,000 Gew.-% (0,200 g) Rhodorsil PI 2074 (Fa. Rhone Poulenc, lodoniumsalz);
- 0,500 Gew.-% (0,050 g) Campherchinon (Fa. Merck, Darmstadt);
- 0,200 Gew.-% (0,020 g) BEDB (Fa. Lambson);

### Herstellung der Vergleichsmischung 2:

Zur Herstellung von 10 g des Vergleichsmischung 2 werden die folgenden Bestandteile intensiv miteinander vermischt:
- 97,300 Gew.-% (9,730 g) 3,4-Epoxycyclohexylmethyl-3,4-epoxycyclohexancarboxylat;
- 2,000 Gew.-% (0,200 g) Rhodorsil PI 2074 (Fa. Rhone Poulenc, lodoniumsalz);
- 0,500 Gew.-% (0,050 g) Campherchinon (Fa. Merck, Darmstadt);
- 0,200 Gew.-% (0,020 g) BEDB (Fa. Lambson);

### Herstellung der Vergleichsmischung 3:

Zur Herstellung von 10 g des Vergleichsmischung 3 werden die folgenden Bestandteile intensiv miteinander vermischt:
- 48,650 Gew.-% (4,865 g) 3,4-Epoxycyclohexylmethyl-3,4-epoxycyclohexancarboxylat;
- 48,650 Gew.-% (4,865 g) 1,3,5,7-Tetrakis-(2,1-ethandiyl-3,4-epoxycyclohexyl)-1,3,5,7-tetramethylcyclotetrasiloxan;
- 2,000 Gew.-% (0,200 g) Rhodorsil PI 2074 (Fa. Rhone Poulenc, lodoniumsalz);
- 0,500 Gew.-% (0,050 g) Campherchinon (Fa. Merck, Darmstadt);
- 0,200 Gew.-% (0,020 g) BEDB (Fa. Lambson);

**Tabelle 1:**

| Haftung der in den Beispielen beschriebenen Adhäsivmischungen: | | |
|---|---|---|
| Adhäsivmischung | Füllungsmaterial | Dentinhaftung [MPa]* |
| Adhäsivmischung 1 | Kationisch härtend | 3,5 |
| Adhäsivmischung 2 | Kationisch härtend | 3,7 |
| Adhäsivmischung 3 | Kationisch härtend | 2,4 |
| Vergleichsmischung 1 | Kationisch härtend | 0,0 |
| Vergleichsmischung 1 | Pertac II (ESPE Dental AG, Seefeld) | 0,0 |
| Vergleichsmischung 2 | Kationisch härtend | 0,0 |
| Vergleichsmischung 2 | Pertac II (ESPE Dental AG, Seefeld) | 0,0 |
| Vergleichsmischung 3 | Kationisch härtend | 0,0 |
| Vergleichsmischung 3 | Pertac II (ESPE Dental AG, Seefeld) | 0,0 |

| | | |
|---|---|---|
| * Mittelwert aus je 5 Messungen | | |

## Patentansprüche

1. Verwendung mindestens einer Komponente i) die fähig ist, eine kationische Polymerisation zu starten und gewählt ist aus der Gruppe ungesättigte Carbonsäuren und deren Anhydriden oder Säurechloriden, ungesättigte organische Phosphorsäuren und deren Estern, ungesättigte organischen Phosphonsäuren und deren Estern, ungesättigte organischen Sulfonsäuren und deren Estern, und mindestens einer Komponente ii), die kationisch polymerisierbar ist, zur Herstellung eines Adhäsivsystems für die Befestigung von kationisch oder radikalisch und kationisch härtenden Materialien auf Wasser enthaltenden Hartgewebe.

2. Verwendung nach Anspruch 1, wobei die Komponente i) in Anteilen von 0,01 bis 95 Gew.-%, und die Komponente ii) in Anteilen von 5 bis 99,99 Gew.-% vorliegt.

3. Verwendung nach einem der vorstehenden Ansprüche, wobei die Materialien Dentalmaterialien und das Hartgewebe Zahn ist.

4. Verwendung nach einem der vorstehenden Ansprüche, wobei mindestens eine der folgenden Komponenten oder eine beliebige Mischung der folgenden Komponenten zusätzlich enthalten ist: iii) ein weiterer radikalischer und/oder kationischer Polymerisationsinitiator oder Mischungen daraus; iv) ein Verdünner; v) ein Füllstoff; vi) eine Fluoridionenquelle; vii) eine Säure, die keine Doppelbindung enthält; viii) ein bakterizid oder antibiotisch wirkendes Mittel.

5. Verwendung nach einem der vorstehenden Ansprüche, wobei die Komponente i) gewählt ist aus:
- ungesättigten Carbonsäuren der Formel: 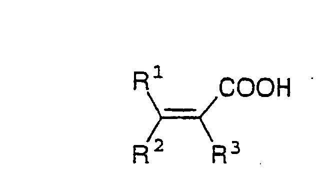 in welcher bedeuten: R¹, R², R³ = H, C₁- bis C₂₅- Alkyl- oder Cycloalkylreste, ggf. substituiert mit N, O, S, Si, P oder Halogen, oder aromatische C₆ bis C₁₂-Reste oder heterocyclische C₃ bis C₁₂-Reste mit N, O, S, P und ggf. substituiert mit Halogen.
- ethylenisch ungesättigte Phosphorsäureester oder Phosphonsäureester der Formel: 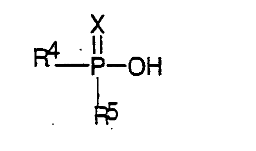 in welcher bedeuten:
X = O oder S; R⁴ und R⁵ unabhängig voneinander H, OH, oder C₁ bis C₂₅-Alkyl oder Cycloalkyl, ggf. substituiert oder verbrückt mit Heteroatomen, wie N, Halogen, Si, O, oder S, aromatische C₆ bis C₁₂ -, oder/und heterocyclische C₄ bis C₁₂-Reste, oder substiuiert mit Acrylsäureestem, wobei die Reste R⁴ und R⁵ auch unabhängig voneinander über O an den Phosphor gebunden sein können,
oder einen Rest der Formel 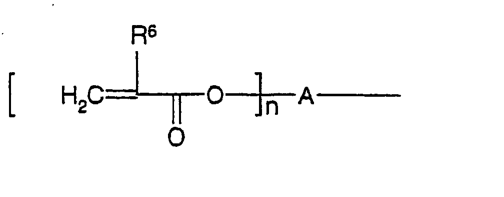
, wobei R⁶ Wasserstoff oder C₁- bis C₆-Alkyl bedeutet und n eine ganze Zahl ≥ 1 ist, und
A ein zweiwertiger C₁- bis C₂₅- Alkyl- oder -Cycloalkylrest ggf. substituiert oder überbrückt mit N, O, S, Si, P oder Halogen, oder aromatische C₆ bis C₁₂-Reste oder heterocyclische C₃ bis C₁₂-Reste mit N, O, S, P und ggf. substituiert mit Halogen, bedeutet, mit der Maßgabe dass der R⁶ enthaltende Rest mindestens einmal vorhanden ist.

6. Verwendung nach einem der vorstehenden Ansprüche, wobei die Mischung durch Zufuhr von elektromagnetischer Strahlung polymerisiert wird, vorzugsweise durch Bestrahlung mit Licht der Wellenlänge von 350 bis 1000 nm.

7. Verwendung nach einem der vorstehenden Ansprüche, wobei unmittelbar nach dem Aufbringen der Mischung die Zahnhartsubstanz mit nur oder auch kationisch polymerisierbarem Material polymerisierbarem Material überschichtet wird.

8. Verwendung nach einem der vorstehenden Ansprüche, wobei vor Aufbringen des Adhäsivsystems die Zahnhartsubstanz nicht geätzt wird.

9. Kit, umfassend a) ein Adhäsivsystem, enthaltend eine Komponente i), die fähig ist, eine kationische Polymerisation zu starten und gewählt ist aus der Gruppe ungesättigte Carbonsäuren und deren Anhydride oder Säurechloride, ungesättigte organische Phosphorsäuren und deren Ester, ungesättigte organischen Phosphonsäuren und deren Ester, ungesättigte organischen Sulfonsäuren und deren Ester, und mindestens einer Komponente ii), die kationisch polymerisierbar ist.

## Claims

1. The use of at least one component i) capable of initiating a cationic polymerization and selected from the group consisting of unsaturated carboxylic acids and their anhydrides or acid chlorides, unsaturated organic phosphoric acids and their esters, unsaturated organic phosphonic acids and their esters, unsaturated organic sulfonic acids and their esters, and at least one component ii) which is cationically polymerizable for producing an adhesive system for affixing cationically or free-radically and cationically curing materials to water-containing hard tissue.

2. The use as claimed in claim 1, wherein component i) is present in fractions of from 0.01 to 95% by weight and component ii) in fractions of from 5 to 99.99% by weight.

3. The use as claimed in one of the above claims, the materials being dental materials and the hard tissue being tooth.

4. The use as claimed in one of the above claims, wherein at least one of the following components or any desired mixture of the following components is additionally present: iii) a further free-radical and/or cationic polymerization initiator or mixtures thereof; iv) a diluent; v) a filler; vi) a f luoride ion source; vii) an acid containing no double bonds; viii) an agent having a bactericidal or antibiotic action.

5. The use as claimed in one of the above claims, wherein component i) is selected from:
- unsaturated carboxylic acids of the formula: 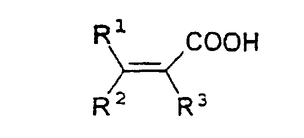 in which R¹, R², R³ = H, C₁ to C₂₅ alkyl or cycloalkyl radicals, unsubstituted or substituted by N, O, S, Si, P or halogen, or aromatic C₆ to C₁₂ radicals or heterocyclic C₃ to C₁₂ radicals with N, O, S, P and unsubstituted or substituted by halogen.
- ethylenically unsaturated phosphoric esters or phosphonic esters of the formula: 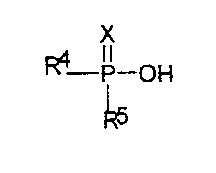
in which:
X = O or S; R⁴ and R⁵ independently of one another are H, OH, or C₁ to C₂₅ alkyl or cycloalkyl, unsubstituted or substituted or bridged by heteroatoms such as N, halogen, Si, O or S, aromatic C₆ to C₁₂ radicals and/or heterocyclic C₄ to C₁₂ radicals, or substituted by acrylic esters, it also being possible for the radicals R⁴ and R⁵ independently of one another to be attached to the phosphorus via O,
or a radical of the formula 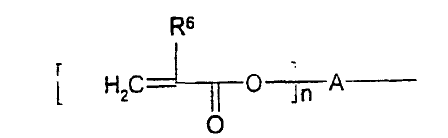 where R⁶ is hydrogen or C₁ to C₆ alkyl and n is an integer ≥ 1, and
A is a divalent C₁ to C₂₅ alkyl or cycloalkyl radical, substituted or unsubstituted or bridged by N, O, S, Si, P or halogen or an aromatic C₆ to C₁₂ radical or heterocyclic C₃ to C₁₂ radical containing N, O, S, or P and unsubstituted or substituted by halogen, with the proviso that the radical that contains R⁶ is present at least once.

6. The use as claimed in one of the above claims, wherein the mixture is polymerized by supplying electromagnetic radiation, preferably by irradiation with light of wavelength from 350 to 1000 nm.

7. The use as claimed in one of the above claims, wherein directly following the application of the mixture the hard tooth substance is overcoated with polymerizable material which can be polymerized only cationically or also cationically.

8. The use as claimed in one of the above claims, wherein the hard tooth substance is not etched before the adhesive system is applied.

9. A kit comprising a) an adhesive system comprising a component i) capable of initiating a cationic polymerization and selected from the group consisting of unsaturated carboxylic acids and their anhydrides or acid chlorides, unsaturated organic phosphoric acids and their esters, unsaturated organic phosphonic acids and their esters, unsaturated organic sulfonic acids and their esters, and at least one component ii) which is cationically polymerizable.

## Revendications

1. Utilisation d'au moins un composant i) qui est apte à amorcer une polymérisation cationique et est choisi dans le groupe des acides carboxyliques insaturés et de leurs anhydrides ou chlorures d'acides, des acides phosphoriques organiques insaturés et de leurs esters, des acides phosphoniques organiques insaturés et de leurs esters, des acides sulfoniques organiques insaturés et de leurs esters, et d'au moins un composant ii) qui est apte à la polymérisation cationique, dans la préparation d'un système adhésif pour la solidification de matériaux durcissables par voie cationique ou radicalaire et cationique sur des tissus durs contenant de l'eau.

2. Utilisation selon la revendication 1, dans laquelle le composant i) est présent en proportions de 0,01 à 95 % en poids et le composant ii) est présent en proportions de 5 à 99,99 % en poids.

3. Utilisation selon l'une quelconque des revendications précédentes, dans laquelle les matériaux sont des matériaux dentaires et le tissu dur est une dent.

4. Utilisation selon l'une quelconque des revendications précédentes, dans laquelle est additionnellement contenu au moins l'un des composants suivants ou un mélange des composants suivants : iii) un autre amorceur de polymérisation cationique et/ou radicalaire
ou des mélanges de ceux-ci ; iv) un diluant ; v) une charge ; vi) une source d'ions fluorure ; vii) un acide qui ne comporte pas de double liaison ; viii) un bactéricide ou un agent à activité antibiotique.

5. Utilisation selon l'une quelconque des revendications précédentes, dans laquelle le composant i) est choisi parmi :
- des acides carboxyliques insaturés de formule : 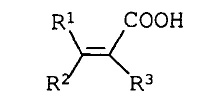 dans laquelle : R¹, R², R³ = H, des radicaux alkyle ou cycloalkyle en C₁ à C₂₅ éventuellement substitués par N, O, S, Si, P ou des atomes d'halogène, ou des radicaux en C₆ à C₁₂ aromatiques ou des radicaux en C₃ à C₁₂ hétérocycliques à N, O, S, P et éventuellement substitués par des atomes d'halogène,
- des esters d'acides phosphoriques ou des esters d'acides phosphoniques, à insaturation éthylénique, de formule : 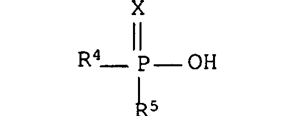 dans laquelle :
X = O ou S ; R⁴ et R⁵ représentent, indépendamment l'un de l'autre, H, OH ou un groupe alkyle ou cycloalkyle en C₁ à C₂₅ éventuellement substitué ou ponté par des hétéroatomes tels que N, halogène, Si, O ou S, des radicaux en C₆ à C₁₂ aromatiques et/ou en C₄ à C₁₂ hétérocycliques, ou substitué par des esters d'acide acrylique, les radicaux R⁴ et R⁵ pouvant également, indépendamment l'un de l'autre, être fixés par O sur le phosphore,
ou un radical de formule 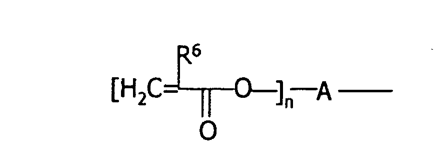 dans laquelle R⁶ représente un atome d'hydrogène ou un groupe alkyle en C₁ à C₆ et n est un nombre entier ≥ 1, et
A représente un radical alkyle ou cycloalkyle en C₁ à C₂₅ éventuellement substitué ou ponté par N, O, S, Si, P ou des atomes d'halogène, ou des radicaux en C₆ à C₁₂ aromatiques ou des radicaux en C₃ à C₁₂ hétérocycliques à N, O, S, P et éventuellement substitués par des atomes d'halogène, étant entendu que le radical contenant R⁶ est présent au moins une fois.

6. Utilisation selon l'une quelconque des revendications précédentes, dans laquelle le mélange est polymérisé par apport d'un rayonnement électromagnétique, de préférence par irradiation avec de la lumière d'une longueur d'onde de 350 à 1 000 nm.

7. Utilisation selon l'une quelconque des revendications précédentes, dans laquelle, immédiatement après l'application du mélange, la substance dure dentaire est revêtue avec du matériau polymérisable par voie seulement ou également cationique.

8. Utilisation selon l'une quelconque des revendications précédentes, dans laquelle, avant l'application du système adhésif, la substance dure dentaire n'est pas attaquée.

9. Nécessaire comprenant a) un système adhésif contenant un composant i) qui est apte à amorcer une polymérisation cationique et est choisi dans le groupe des acides carboxyliques insaturés et de leurs anhydrides ou chlorures d'acides, des acides phosphoriques organiques insaturés et de leurs esters, des acides phosphoniques organiques insaturés et de leurs esters, des acides sulfoniques organiques insaturés et de leurs esters, et au moins un composant ii) qui est apte à la polymérisation cationique.
